# EUROPEAN PATENT SPECIFICATION

(11) **EP 1 352 018 B1**
(45) Date of publication and mention of the grant of the patent: **05.12.2007**
(21) Application number: 02712822.2
(22) Date of filing: 16.01.2002
(51) Int. Cl.: C08J 7/04, C08J 7/12, C08J 7/18, C12N 11/08, A61L 27/34, A61L 29/08, A61L 31/10

(54) **SURFACE PASSIVATION OF ORGANIC POLYMERS AND ELASTOMERS**
OBERFLÄCHENPASSIVIERUNG VON ORGANISCHEN KUNSTSTOFFEN
PASSIVATION SUPERFICIELLE DES POLYMERES ET DES ELASTOMERES ORGANIQUES

(30) Priority: 16.01.2001 GB 0101126
(43) Date of publication of application: 15.10.2003
(73) Proprietor: CSEM Centre Suisse d'Electronique et de Microtechnique SA, 2007 Neuchâtel (CH)
(72) Inventor: SIGRIST, Hans, CH-3309 Kernelried (CH); LINDER, Vincent, CH-6003 Luzern (CH)
(74) Representative: Thornton, Neil
(86) International application number: PCT/EP2002/000386
(87) International publication number: WO 2002/055593

(56) References cited:
- WO-A-91/16425
- WO-A-99/15917
- DE-A- 19 724 869
- DE-A- 19 818 360
- US-A- 4 326 532
- US-A- 5 714 360
- PATENT ABSTRACTS OF JAPAN vol. 011, no. 307 (C-450), 7 October 1987 (1987-10-07) & JP 62 097611 A (MITSUBISHI RAYON CO LTD), 7 May 1987 (1987-05-07)

## Description

### Prior art

It is known that the properties of polymer surfaces may be modulated by integrating polar or charged components or monomeric precursor analogues in the polymerization process. Post-polymerization surface treatments are equally established, e.g. plasma treatment followed by exposure to aqueous media renders organic polymers highly wettable, and surface silylation has been applied to introduce selectively reactive functionalities on organic polymer material surfaces. In a recent approach, PDMS (polydimethylsiloxane) microchannels were fabricated using replica molding techniques. Surface passivation was achieved by multimeric protein (Immunoglobulin M) adsorption or by theromochemical crosslinking of a capture protein (protein A) with glutaraldehyde. (E. Eteshola, D. Leckband, Sensors and Actuators B 72 (2001) 129-133).

The increasing use of binary or ternary composite polymers in bio-analytical and medical devices demands unifying surface modification processes that are indistinguishably applicable to different polymer chemistries. It is widely documented that C-H, C=C and O-H bonds - the most frequent chemical bonds in organic polymers and elastomers - are chemically reactive with generated carbenes and ketyl radicals. Such chemical intermediates can be generated locally by thermal or light activation. The reactivity of many organic polymers with e.g. photogenerated carbenes was shown using low molecular weight crosslinkers or photolabel derivatized macromolecules including proteins and nucleic acids.

Several patents and scientific research articles report on the advantageous properties of oligo- and polysaccharides to adsorb and store water or aqueous media. When conjugated to material surfaces polysaccharides form biocompatible passivating layers leading to improved performance of bioanalytical systems. However, establishment of such passivating layers requires a specific substrate and a sequence of at least two surface chemical steps to attain the requested conjugate.

### Summary of the Invention

The invention in its various aspects is defined in the appended independent claims, to which reference should now be made. Preferred or advantageous features of the invention are set out in dependent subclaims.

Thus, the invention may advantageously overcome the problems in the prior art discussed above.

In its various aspects, the invention relates to the treatment of material surfaces, in particular organic polymers and elastomers of natural or synthetic origin that require unique or particular surface properties for adequate device function. Although organic polymers are numerous with respect to their chemistry, complexity, physical properties, their mode of use and fields of application, there are restricted means to render them compatible with biological systems. In view of engineering of material surfaces for bioanalytical purposes, surface structuring with biomolecules, and medical applications there is a need for adaptation of organic materials and composites for appropriate function when contacted with biological systems. This invention relates to material surface treatment that may advantageously result in a close mimic of biological systems: the generic approach of in situ generation of chemically reactive species in conjunction with the passivating properties of polysaccharide may advantageously render novel properties to materials and may thus improve the efficiency of biocompatible devices.

The surfaces of solid organic polymers and elastomers are prone to physical adsorption and bi-directional diffusion of low molecular weight molecules. Diffusive sorption and release processes, as well as adsorptive binding properties of polymeric materials may advantageously be drastically suppressed by treatment of the polymer surfaces with oligo- or polysaccharides. Surface passivation by covalent or adsorptive thin-film coating may be attained by depositing and subsequent immobilization of biopolymers onto organic polymer or elastomer surfaces. Preferably, the passivating material is covalently bonded to the organic material yielding biocompatible surfaces as used in medical and analytical applications. With aryldiazirine or benzophenone derivatized oligo- or polysaccharides, covalent surface passivation may be attained by photo- or thermal activation of the polysaccharide derivative. Surface passivation is exemplified by polydimethyldioxane microchannel treatment and application of the resulting systems for the detection of immunointeractions.

### Description of the invention

The implementation of the invention will now be described, including description of several embodiments. Further details are also set out in the accompanying drawings. In a preferred embodiment, the invention may provide a surprisingly simple process to passivate organic polymer or elastomer surfaces. Thus, in tests, different types of polymer materials, individually or as composite material, were passivated by depositing carbene- or ketyl radical forming derivatives of polysaccharides in target surfaces. Derivatives of aminated polysaccharides such as aminodextran or chitosan were prepared: the polysaccharides were thermochemically modified with benzophenone-4-isothiocyanate or isothiocyano-aryl-diazirines, yielding benzophenone-dextran and benzophenone-chitosan, or aryldiazirine-dextran and aryldiazirine-chitosan, respectively. For covalent immobilization of these passivating agents, the reagents were thin-film deposited on the organic polymer surfaces and irradiated with activating light (wavelength: 350+20 nanometer, power: 10 microwatts per square centremetre, exposure time: 4 mintutes) or by heating to 80-115°C. Such surface treatment was effective for passivation of PDMS (polydimethylsiloxane) elastomers and polymers as for example, parylene, polystyrene, polyurethane, polycarbonate, polyvinylalcohol or polyvinyl difluoride. For analytical purposes, PDMS microchannels, forming parts of microfluidic systems, were passivated by either protein-mediated binding of dextran, or by direct immobilization of radical or carbene generating polysaccharides on PDMS.

Immobilization was feasible ex situ and in situ, in particular after the formation of functional microchannels. Both passivation procedures yielded surfaces that prevented diffusion of charged or polar low molecular weight chemicals into the PDMS matrix and suppressed the adsorption of proteins e.g. immunoglobulins to analytically non-interfering levels. Furthermore, attachment of immunoreagents to passivated microfluidic channels or organic polymer surfaces via secondary functions (e.g. binding of antigens to photoimmobilized carboxy-dextran) generated immunoreactive polymer or elastomer surfaces. The described surface treatment may thus open new routes to fast and simply-implemented in situ passivation of structured organic materials, making them available for bioanalytical and biomedical applications.

### Surface Bio-Passivation of Replicated µ-Fluidic Channels

Polydimethylsiloxane (PDMS) appeared recently as a material of choice for rapid and accurate replication of polymer-based microfluidic networks. However, due to its hydrophobicity, the surface strongly interacts with apolar analytes or species containing apolar domains, resulting in significant loss of sample to the substrate and, consequently, poor analytical performance. This contribution describes, with reference to the accompanying drawings and figure legends, the characterization of a native PDMS surface passivation treatment in microchannels.

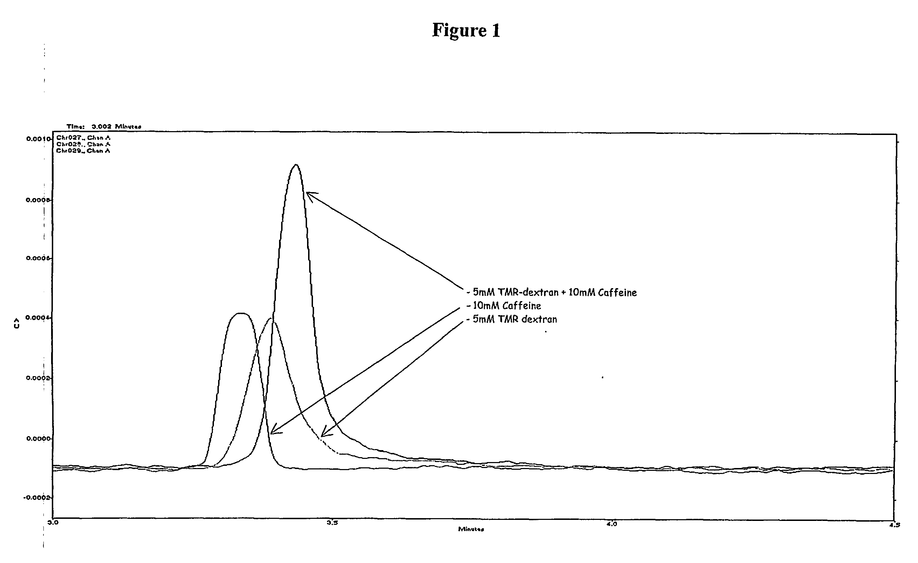

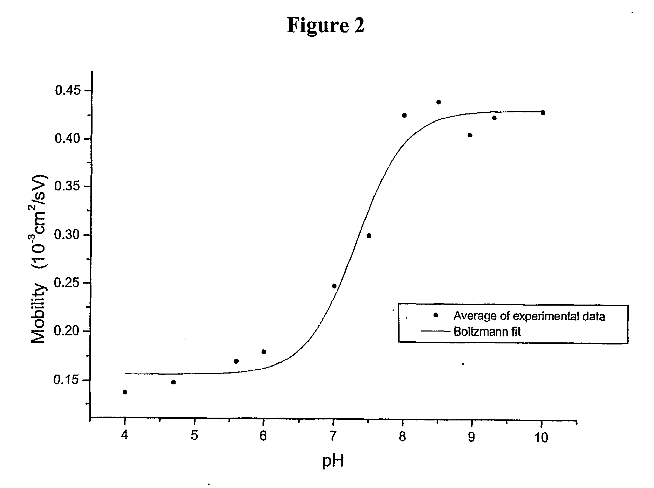

Figures 1 and 2 show the behaviour of a fluorescent neutral marker in fused-silica/Pyrex;

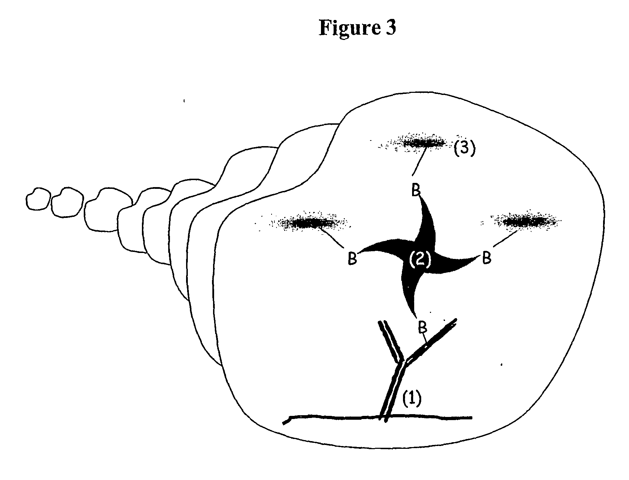

Figure 3 shows schematically PDMS surface bio-passivation;

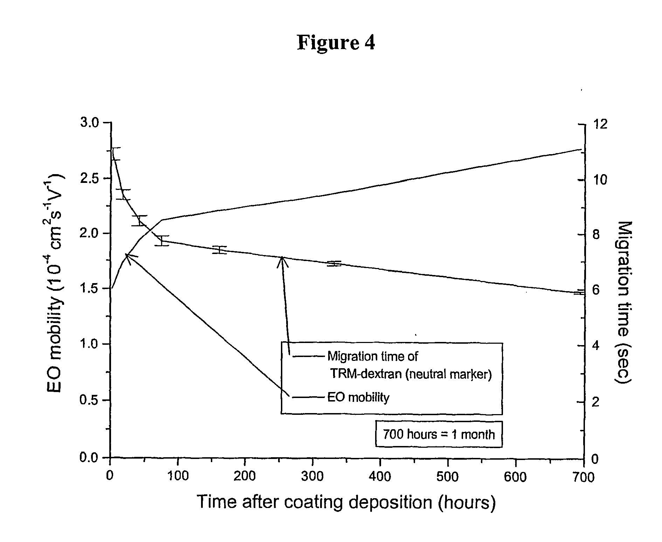

Figure 4 shows EO mobility in coated channels;

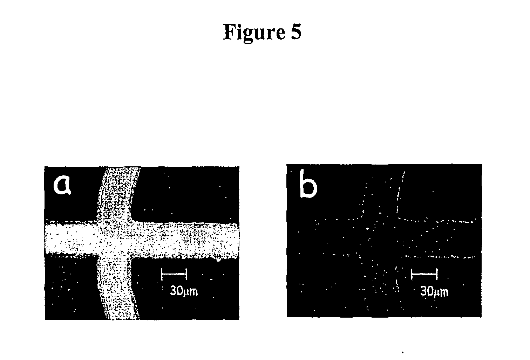

Figure 5 shows passivation and coating stability; and

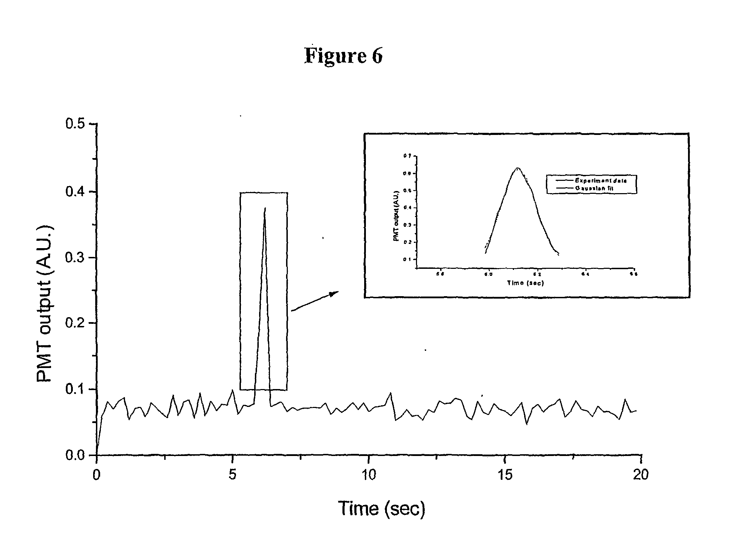

Figure 6 shows biomolecule mobility in coated channels.

### Figure Legends

### Figure 1

Comparative capillary zone electrophoresis (CZE) runs of TMR-dextran and/or Caffeine.
Samples: in Na-acetate buffer 30mM pH=4.7
Working buffer: 30mM Na-acetate pH=4.7
Instrument: P/ACE 5510, UV detection at 214 nm
Exp. Conditions: 6kV, 5sec injection time, 25°C
Capillary: 20/27cm, ID=50 µm.

Tetramethylrhodamine labeled dextran 70 KDa (*TMR-dextran*) was shown to be neutral over the pH range 4.7 to 9.3. CZE runs of both caffeine (UV neutral marker) and TMR-dextran gave identical migration times.

### Figure 2

Boltzman fit for EO mobility measurements in Pyrex channels using TMR-dextran.
Pyrex channel, HF etched (20 µm deep)
Separation channel: total length 6cm, 70µm wide
All buffers ionic strength I = 14mM
E = 650 Vcm⁻¹ (buffers pH 4.0 to 8.5)
E = 430 Vcm⁻¹ (buffers pH 8.95 to 10)
LIF detection (488 nm excitation)

### Pinched injection

TMR-dextran was used as a fluorescent marker for EO mobility determination in Pyrex channels with a selection of acetate, phosphate and tetraborate buffers ranging from pH 4.0 to 10. Ionic strength remained constant. Results compare well with published data.

### Figure 3

Schematic diagram of the coating used for passivation. (1) Physisorption of biotin conjugate of IgG to PDMS; (2) Neutravidin; (3) Biotin conjugate of dextran 10kDa.

### Figure 4

Migration time of TMR-dextran in coated channels and calculated EO mobility.

Experimental conditions: running buffer (RB) 6.74 mM Tetraborate buffer pH 9.3; sample 50mM TMR-dextran in RB; E=825 Vcm⁻¹; LIF detection (488nm excitation), pinched injection.

EO mobility was measured in microchannels by monitoring TMR-dextran detection times. Good reproducibility was obtained (RSD (n=10) <2%). However, EO mobility doubled within one month; this variation requires the use of an internal standard.

### Figure 5

Adsorption of 20mM BODIPY-Digoxigenin in (a) uncoated and (b) coated channels (2 minutes incubation, hydrodynamic flow).

Channel preparation: replicas were obtained by moulding PDMS using a micromachined Si wafer as master. Drilled PDMS slabs were sealed against Pyrex wafers.

The three layer coating dramatically decreased adsorption for a variety of fluorescently labeled molecules and biopolymers, such as BODIPY-digoxigenin (MW 0.8kDa), TMR-dextran (MW 70kDa), FITC-human IgG (MW 150 kDa), and others.

The three layer coating appeared to be stable upon extensive exposure to buffers of neutral to basic pH, urine and human blood plasma. Short exposure to weak detergents (such as Tween 20) may be considered, but strong detergents (SDS) and very basic solutions (NaOH 0.1M) damage the coating rapidly.

### Figure 6

Recorded peak compared with its Gaussian fit (zoom). Experimental conditions: working buffer 7.92 mM Na-phosphate buffer pH 7.0; sample 5µg/ml fluorescein mouse-IgG in diluted PBS; E=453 Vcm⁻¹; LIF detection (488nm excitation), pinched injection. Full run recorded at 5Hz, peak (zoom) at 500Hz.

Fluorescein-labeled mouse-IgG can be analyzed by CZE in a coated channel. The Gaussian peaks that are recorded indicate that no significant adsorption occurs. In uncoated channels, IgG adsorbs to the elastomer surface: it does not reach the detector.

### Conclusion

The protein-based surface modification considerably decreases adsorption of fluorescently labelled low and high molecular weight substances. Moreover, the passivation layer is stable in buffers, as well as in biological fluids such as serum or urine. Electroosmotic pumping in modified channels is also possible, making this an attractive surface treatment approach for many analytical applications.

## Claims

1. Surface treatment for passivating organic polymer material surfaces with polysaccharides as passivating agents, wherein the passivating agent contains at least one primary functional group which is substituted with a reagent, such as benzophenone-4-isothiocyanate or with diazirino-aryl-isothiocyanates, and said reagent is converted into a reactive species by heating it to a temperature of 80-115°C, said reactive species thus achieving covalent binding of the passivating agent to said organic polymer material surfaces.

2. Surface treatment according to claim 1, wherein said organic polymer material is prepared by chemical precursor polymerization or is a material of natural origin.

3. Surface treatment according to claim 1 or claim 2, wherein said organic polymer material surfaces are microstructured.

4. Surface treatment according to any preceding claim, wherein the polysaccharides are aminodextran or chitosan.

5. Surface treatment according to any preceding claim, wherein the passivating agent carries one or more secondary functional groups that allow covalent binding of probe molecules and/or receptors.

6. Surface treatment according to claim 5, wherein said secondary functional groups are amino groups, carboxyl groups, maleimides, thiols, biotin, epoxides, chelating- or photoreactive entities.

7. Application of the surface treatment according to any of claims 1 to 6 for use in microcontact printing or for treating medical devices, biosensor platforms or fluidic devices.

## Patentansprüche

1. Oberflächenbehandlung zum Passivieren von Oberflächen aus organischem Polymermaterial mit Polysacchariden als Passivierungsmittel, wobei das Passivierungsmittel wenigstens eine primäre funktionelle Gruppe enthält, die substituiert ist durch ein Reagens wie Benzophenon-4-isothiocyanat oder durch Diazirino-aryl-isothiocyanat, und das genannte Reagens durch Erhitzen auf eine Temperatur von 80-115°C in eine reaktive Spezies umgewandelt wird, wobei die genannte reaktive Spezies so eine kovalente Bindung des Passivierungsmittels an die genannten Oberflächen aus organischem Polymermaterial erreicht.

2. Oberflächenbehandlung nach Anspruch 1, wobei das genannte organische Polymermaterial durch chemische Vorläuferpolymerisation hergestellt wird oder ein Material natürlichen Ursprungs ist.

3. Oberflächenbehandlung nach Anspruch 1 oder Anspruch 2, wobei die genannten Oberflächen aus organischem Polymermaterial eine Mikrostruktur haben.

4. Oberflächenbehandlung nach einem der vorherigen Ansprüche, wobei die Polysaccharide Aminodextran oder Chitosan sind.

5. Oberflächenbehandlung nach einem der vorherigen Ansprüche, wobei das Passivierungsmittel eine oder mehrere sekundäre funktionelle Gruppen beinhaltet, die eine kovalente Bindung von Sondenmolekülen und/oder Rezeptoren ermöglichen.

6. Oberflächenbehandlung nach Anspruch 5, wobei die genannten sekundären funktionellen Gruppen Aminogruppen, Carboxylgruppen, Maleimide, Thiole, Biotin, Epoxide, chelatbildende oder photoreaktive Entitäten sind.

7. Anwendung der Oberflächenbehandlung nach einem der Ansprüche 1 bis 6 zur Verwendung beim Mikrokontaktdrucken oder zum Behandeln von medizinischen Vorrichtungen, Biosensorplattformen oder fluidischen Geräten.

## Revendications

1. Traitement de surface pour la passivation de surfaces d'un matériau polymère organique utilisant des polysaccharides comme agents de passivation, où l'agent de passivation contient au moins un groupement fonctionnel primaire qui est substitué par un réactif tel que le benzophénone-4-isothiocyanate ou par des isothiocyanates d'aryle à radical diazirino, et où ledit réactif est converti en des espèces réactives par chauffage à une température de 80-115 °C, lesdites espèces réactives produisant ainsi une liaison covalente de l'agent de passivation auxdites surfaces d'un matériau polymère organique.

2. Traitement de surface selon la revendication 1, où ledit matériau polymère organique est préparé par polymérisation d'un précurseur chimique ou est un matériau d'origine naturelle.

3. Traitement de surface selon la revendication 1 ou la revendication 2, où les surfaces d'un matériau polymère organique sont microstructurées.

4. Traitement de surface selon l'une quelconque des revendications précédentes, où les polysaccharides sont un aminodextrane ou un chitosane.

5. Traitement de surface selon l'une quelconque des revendications précédentes, où l'agent de passivation porte un ou plusieurs groupements fonctionnels secondaires qui permettent la liaison à des molécules sondes et/ou récepteurs.

6. Traitement de surface selon la revendication 5, où lesdits groupements fonctionnels secondaires sont des groupements aminés, des groupements carboxyles, des maléimides, des thiols, la biotine, des époxydes ou des entités chélatantes ou photoréactives.

7. Application du traitement de surface selon l'une quelconque des revendications 1 à 6 dans des usages qui se rapportent à l'impression par microcontact ou au traitement de dispositifs médicaux, de plateformes de biocapteurs ou de systèmes fluidiques.
